(19) Europäisches Patentamt
European Patent Office
Office européen des brevets

(11) Publication number: **0 107 652**
**B1**

(12) **EUROPEAN PATENT SPECIFICATION**

(45) Date of publication of patent specification: **29.07.87**

(51) Int. Cl.$^4$: **C 12 P 19/24,** C 07 H 3/08

(21) Application number: **82901758.1**

(22) Date of filing: **23.04.82**

(86) International application number:
**PCT/US82/00534**

(87) International publication number:
**WO 83/03846 10.11.83 Gazette 83/26**

# (54) PREPARATION OF 6-DEOXY-D-FRUCTOSE AND 6-DEOXY-L-SORBOSE.

(43) Date of publication of application:
**09.05.84 Bulletin 84/19**

(45) Publication of the grant of the patent:
**29.07.87 Bulletin 87/31**

(84) Designated Contracting States:
**CH DE FR GB LI NL**

(56) References cited:

**CARBOHYDRATE RESEARCH, vol. 80, Mai 6, 1980, pages 215-222, Elsevier Scientific Publishing Co., Amsterdam, NL; S. MORGENLIE: "Gaschromatography-mass spectrometry of hexuloses and pentuloses as their O-isopropylidene derivatives: analysis of product mixtures from triose aldol-condensations"**

**BIOCHEMISTRY, vol. 8, no. 1, January 1969, pages 98-108; T.H. CHIU et al.: "L-rhamnulose 1-phosphate aldolase from Escherichia coli. Crystallization and properties"**

(73) Proprietor: **MASSACHUSETTS INSTITUTE OF TECHNOLOGY**
**77 Massachusetts Avenue**
**Cambridge, MA 02139 (US)**
(73) Proprietor: **FIRMENICH SA**
**Case postale 239**
**CH-1211 Genève 9 (CH)**

(72) Inventor: **WHITESIDES, George M.**
**124 Grasmere Street**
**Newton, MA 02160 (US)**
Inventor: **MAZENOD, François P.**
**Firmenich, S.A. Case postale 239**
**CH-11211 Geneve 8 (CH)**
Inventor: **WONG, Chi-Wuei**
**Department of Chemistry Texas A & M University**
**College Station TX 77843 (US)**

(74) Representative: **Salvadori, Giuseppe, Dr.**
**c/o Firmenich S.A. Case Postale 239**
**CH-1211 Genève 8 (CH)**

**EP 0 107 652 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

JOURNAL OF ORGANIC CHEMISTRY, vol. 48, October 7, 1983, pages 3493-3497, American Chemical Society; C.-H. WONG et al.: "Chemical and enzymatic syntheses of 6-deoxyhexoses. Conversion to 2,5-dimethyl-4-hydroxy-2,3-dihydrofuran-3-one (Furaneol) and analogues"

N, CHEMICAL ABSTRACTS, VOLUME 68, ISSUED 26 FEBRUARY 1968 (COLUMBUS, OHIO, U.S.A.), H. KAUFMANN ET AL "DEOXY SUGARS. XLI 6-DEOXY-L-IDOSE, 6-DEOXY-L-SORBOSE, AND 6-DEOXY-L-PSICOSE", SEE PAGE 3898, COLUMN 1, THE ABSTRACT NUMBER 39963v, HELV. CHIM. ACTA 50(8), 2280-7(1967)(GER.)

N, CHEMICAL ABSTRACTS, VOLUME 91, ISSUED 16 JULY 1979 (COLUMBUS, OHIO, U.S.A.), M. MATSUI ET AL "2,5-DIMETHYL-4-HYDROXY-2,3-DIHYDROFURAN-3-ONE", SEE PAGE 637, COLUMN 2, THE ABSTRACT NUMBER 20309q, JPN. KOKAI TOKKYO KOHO 79 19,962 15 FEBRUARY 1979

**Description**

Technical Field

This invention relates to organic chemical synthesis and, in particular, synthesis of 6-deoxy sugars.

Background of the Invention

The 6-deoxy sugars constitute particularly useful starting materials for the preparation of 4-hydroxy-2,5-dimethyl-2,3-dihyderofuran-3-one, better known by the name of FURANEOL[R] (registered trademark of Firmenich SA, Geneva). M. Matsui et al. [see Chem. Abstr., *91*, 2030q (1979)], for example, have described the method for converting 6-deoxy-D-glucose into FURANEOL. Although the yields observed during this conversion were very satisfactory, the method of Matsui suffers from a major drawback, i.e., the high cost of the starting material whose preparation requires four reaction steps. This invention concerns an original solution to this problem. We have found that 6-deoxy-D-fructose and 6-deoxy-L-sorbose, of the following formulas respectively

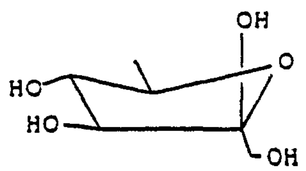

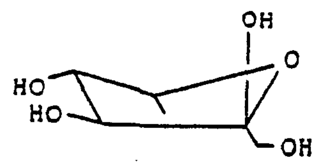

can be converted into FURANEOL in excellent yield, with the conversion taking place in the presence of an organic nitrogen base and a carboxylic acid, under reaction conditions similar to those described by Matsui.

Summary of the Invention

This invention concerns a process for the preparation of 6-deoxy-D-fructose and/or 6-deoxy-L-sorbose characterized by the fact that the following steps are carried out consecutively:

a. Treatment of fructrose-1.6-diphosphate with D- and/or L-lactaldehyde in the presence of an enzymatic system composed of aldolase and triose phosphate isomerase in aqueous medium at pH 7.0, and

b. Hydrolysis of the monophosphate salt thus obtained. This invention also concerns a process for the preparation of 6-deoxy-D-fructose and/or 6-deoxy-L-sorbose which consists of treating, 1,3-dihydroxyacetone phosphate of the formula

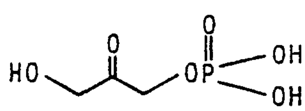

with D- and/or L-lactaldehyde in the presence of an enzymatic system composed of aldolase and triose phosphate isomerase in aqueous medium at pH 7.0, and hydrolyzing the monophosphate salt thus obtained.

The processes described above can be illustrated by the reaction scheme below:

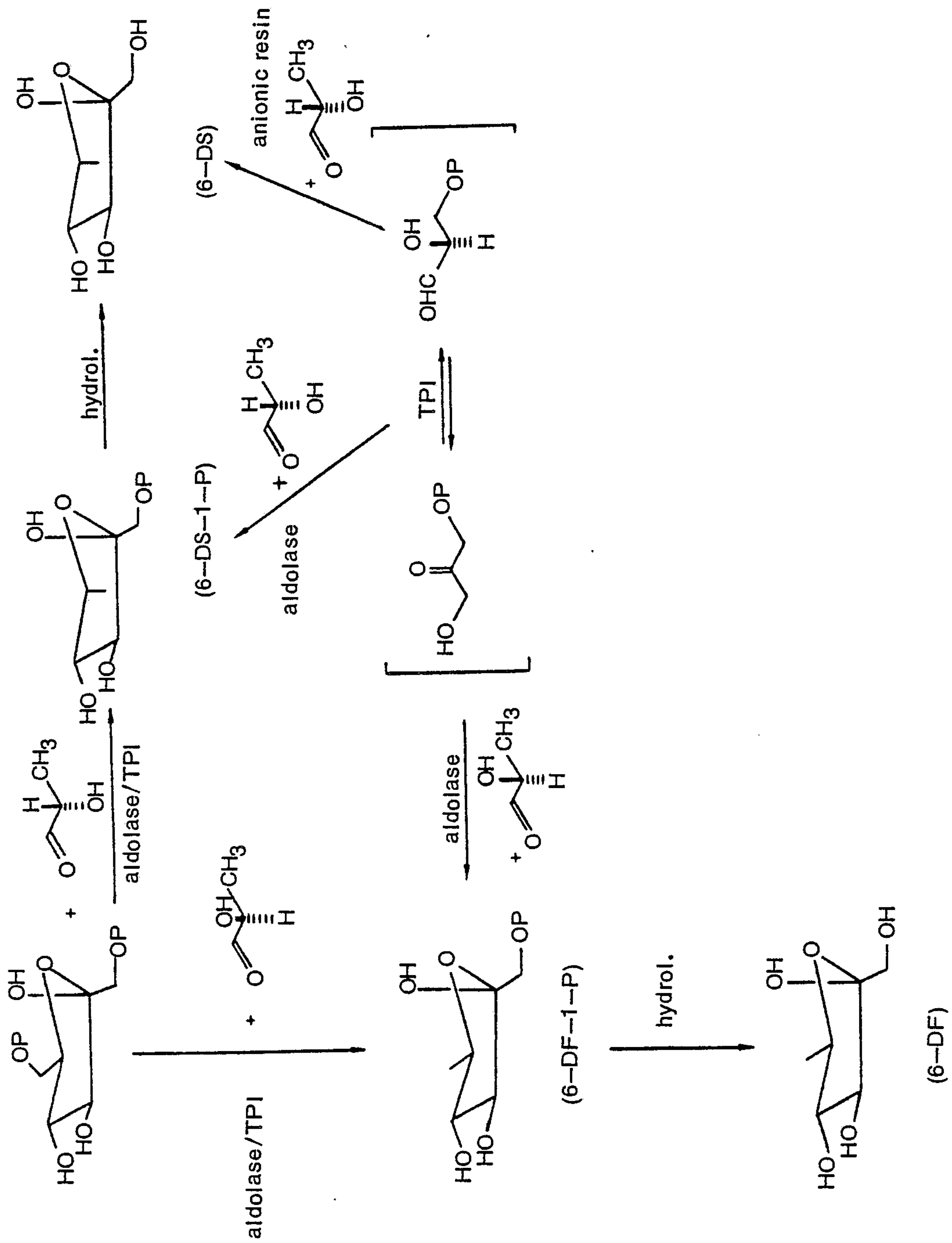
aldolase/TPI
aldolase/TPI
hydrol.
(6-DS-1-P)
aldolase
TPI
(6-DS)
anionic resin
aldolase
(6-DF-1-P)
hydrol.
(6-DF)

Definition of abbreviations:
6—DF = deoxyfructose; 6—DF—1—P = 6-deoxyfructose-1-phosphate
6—DS = 6-deoxysorbose; 6—DS—1—P = 6-Deoxysorbose-1-phosphate
TPI = triose phosphate isomerase

The first step of the procedures making use of aldolase and triose phosphate isomerase (TPI) formally constitutes an example of aldol condensation. This reaction proceeds in a perfectly selective manner and does not require the protection of other functional groups present in the molecule. The required reaction conditions are also particularly mild (pH 7.0 — room temperature), and it follows that the industrial application of the aforementioned procedures for the preparation of 6-deoxy-D-fructose or 6-deoxy-L-sorbose can be visualized directly. The enzymatic system chosen was immobilized on a solid support in accordance with the method described in J. Am. Chem. Soc., *102*, 6324 (1980). The aldolase and the triose phosphate isomerase are commerical enzymes.

The D- and L-lactaldehyde were prepared from L- and D-threonine by reaction with ninhydrin according to the method described in J. Biol. Chem., *241*, 3028—35 (1966).

The 1,3-dihydroxyacetone phosphate (DHAP) can be obtained either by enzymatic phosphorylation of 1,3-dihydroxyacetone (DHA) using ATP and the monopotassium salt of phosphoenolpyruvate, or by the chemical phosphorylation of the same DHA using $POCl_3$. Fructose-1,6-diphosphate, in the form of its sodium or dicalcium salt, is a commercial product. If necessary, it can be purified before its use by a treatment using an anionic exchange resin, for example, of the type DOWEX 1. The particular purification method used is described in detail in the examples which follow.

The deoxyfuranoses prepared in accordance with the procedures described above can be used directly for their conversion into FURANEOL.

Description of the Preferred Embodiments

The invention is illustrated in a more detailed manner by the following examples, in which the temperatures are indicated in degrees Centigrade and the abbreviations have the meanings customary in the art.

Example 1

a. *6-Deoxy-D-fructose-1-phosphate* (6—DF—1—P) 100 ml of an aqueous solution containing fructose-1.6-diphosphate (200 mmoles; solution prepared from its barium salt by treatment with a type DOWEX 50 anionic exchange resin and neutralization with a solution of NaOH) and 25 mmoles of D-lactaldehyde (pH = 7.0) was deoxygenated with argon.

An enzymatic system composed FDP-aldolase (500 U, 15 ml of gel) and triose phosphate isomerase (200 U, 1 ml of gel) coimmobilized on a solid support of polyacrylamide was added to the aqueous solution of FDP at room temperature and with vigorous agitation. The progress of the reaction was followed by enzymatic analysis. The reaction took 2—1/2 days. After separation of the gel, the solution was mixed with 45 mmoles of $BaCl_2$ and the pH was adjusted to 7.8 at 4°-. At this temperature, 1 liter of acetone was added, which induced the formation of a precipitate (14.8 g) containing 34 mmoles (yield 87%) of the barium salt of 6-deoxy-D-fructose-1-phosphate (6-DF-1-$PBa_2$). The enzymatic activities of the recovered aldolase and isomerase were 78% and 75%, respectively.
NMR(250 MHz, $D_2O$): 1.33 and 1.35 (3H, d); 3.6—4.2 (5H, m) δ ppm.

b. *6-Deoxy-L-sorbose-1-phosphate* (6—DS—1—P) This compound was prepared in accordance with the procedure described under letter a., using L-lactaldehyde. The yield obtained was 80% of a product with a purity of 86%.
NMR(250 MHz, $D_2O$): 1.18 and 1.29 (3H, d); 3.4—4.5 (5H, m) δ ppm.

c. *(6—DF—1—P) and (6—DS—1P* The mixtures containing (6—DF—1—P) and (6—DS—1—P) at the same time were prepared by the procedure described under letter a. above, starting with 0.2 moles of fructose-1.6-diphosphate and 0.3 moles of DL-lactaldehyde and in the presence of 25 ml of aldolase gel (100 U) and 3 ml of triose phosphate isomerase gel (500 U). The product was isolated in the form of the barium salt (152 g; 328 mmoles) with a purity of 82% (yield 82%).

The 6-deoxyfructose and the 6-deoxysorbose are prepared from the respective phosphates as follows:

10 mmoles of the phosphate salt obtained as indicated above was suspended in 50 ml of water and the mixture was treated with 10 g of a DOWEX 50 acid exchange resin. The acid solution obtained (pH 1.0), after filtration, was heated at 90° for 8 h, and then a basic DOWEX 1 exchange resin was added until the solution reached a pH of approximately 6—7 at 25°. The mixture was then filtered and the clear filtrate was concentrated to eliminate the water present. A mixture of methanol (10 ml) and acetone (10 ml) was added to the residue and the precipitate which resulted from this was separated. The clear solution was then concentrated until an oily residue was obtained, composed of the desired deoxy-sugar.
*6-Deoxy-D-fructose*($D_2O$,DSS,250 MHz): 1.32(3H,d,J=6.1); 3.32 (1H, q);
3.65—3.88 (2H,m); 4.06 (1H,m); 4.88 (1H) δ ppm;
6-Deoxy-L-sorbose ($D_2O$, DSS 250 MHz): 1.16 (3H, d, J=6.7); 1.27 (3H, d, J=6.1);
3.55 (2H, d, J=4.9); 4.05 (1H, d, J=4.3); 4.18 (1H, m); 4.38 (1H, m); 4.84 (1H) δ ppm.

The barium salt of fructose-1,6-diphosphate, used as the starting material in the procedure described above, was prepared as follows:

20 g of the dicalcium salt of fructose-1,6-diphosphate (commercial product with a purity of 63%) in 300 ml of water was treated with 400 g of type DOWEX 50 acid exchange resin. After filtration and washing with 100 ml of water, the solution was passed through a sintered glass filter containing 400 g of a basic DOWEX 1 exchange resin. It was then washed with dilute HCl (2.5 liters). 90 mmoles of barium chloride was added to the solution obtained, the pH of which was brought to 8.0 by the addition of a solution of NaOH. 500 ml of ethanol was then added to the solution cooled to 4°, and 28 g of the precipitate thus formed was collected by filtration. An enzymatic analysis using aldolase and glycerol phosphate dehydrogenase indicates a content of 86% of the barium salt of F—1.6 diphosphate (F—1.6—$DPBa_2$).

**Claims**

1. Process for the preparation of 6-deoxy-D-fructose and/or 6-deoxy-L-sorbose, characterized by the fact that

a. Fructose-1,6-diphosphate or 1,3-dihydroxyacetone phosphate of the formula

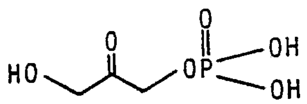

is treated with D- and/or L-lactaldehyde in the presence of an enzymatic system composed of aldolase and triose phosphate isomerase in aqueous medium at pH 7.0, and

b, The monophosphate salt thus obtained is hydrolyzed.

2. Process pursuant to Claim 1, characterized by the fact that the aldolase and the triose phosphate isomerase used are coimmobilized on a solid support.

**Patentansprüche**

1. Verfahren zur Herstellung von 6-Deoxy-D-fructose und/oder 6-Deoxy-L-sorbose, dadurch gekennzeichnet, dass man

a. Fructose-1,6-diphosphat oder 1,3-Dihydroxy-aceton-phosphat der Formel

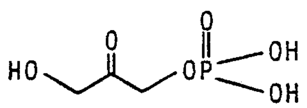

mit D- und/oder L-acetaldehyd in Ansesenheit eines aus Aldolase und Triosephosphat Isomerase bestehenden enzymatischen Systems in einem wässrigen Milieu zu pH 7,0 umsetzt, und

b. das so erhaltene Monophosphat-Salz hydrolisiert.

2. Verfahren gemäss Anspruch 1, dadurch gekennzeichnet, dass Aldolase und Triose-phosphat Isomerase auf dem selben Träger immobilisiert sind.

**Revendications**

1. Procédé pour la préparation de 6-désoxy-D-fructose et/ou 6-désoxy-L-sorbose, caractérisé en ce que

a. le fructose-1,6-diphosphate ou le phosphate de 1,3-dihydroxy-acé tone de formule

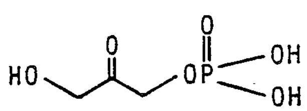

est traité avec le D- et/ou L-lactaldéhyde en présence d'un système enzymatique composé d'adolase et triose phosphate isomérase en milieu aqueux à pH 7,0 et

b. le sel monophophorique ainsi obtenu est hydrolysé.

2. Procédé selon la revendication 1, caractérisé en ce que l'adolase et la triose-phosphate isomérase emloyé sont coimmobilisées sur un support solide.